Europäisches Patentamt

European Patent Office

Office européen des brevets

(11) Publication number: **0 147 048**
**B1**

# EUROPEAN PATENT SPECIFICATION

(45) Date of publication of patent specification: **31.01.90**

(51) Int. Cl.⁵: **A 61 F 5/451**, A 61 F 5/44

(21) Application number: **84307940.1**

(22) Date of filing: **15.11.84**

(54) **Urine drainage bag.**

(30) Priority: **29.11.83 GB 8331797**

(43) Date of publication of application:
**03.07.85 Bulletin 85/27**

(45) Publication of the grant of the patent:
**31.01.90 Bulletin 90/05**

(84) Designated Contracting States:
**AT BE CH DE FR IT LI LU NL SE**

(56) References cited:
**DE-U-7 033 827**
**GB-A-1 319 442**
**GB-A-1 382 014**
**GB-A-2 080 247**
**US-A-3 529 599**
**US-A-3 568 965**
**US-A-3 650 272**

(73) Proprietor: **E.R. Squibb & Sons, Inc.**
**Lawrenceville-Princeton Road**
**Princeton, N.J. 08540-4000 (US)**

(72) Inventor: **Steer, Peter Leslie**
**Woodlands Rise Kingscote**
**East Grinstead Sussex (GB)**

(74) Representative: **Cook, Anthony John et al**
**D. YOUNG & CO. 10, Staple Inn**
**London, WC1V 7RD (GB)**

Courier Press, Leamington Spa, England.

# Description

This invention relates to a method of making a urine drainage bag.

There have been numerous prior designs of drainage bags and problems have arisen in making an effective and durable seal where the inlet tube and outlet tap are connected to the bag. For example in bags such as are shown in British Patent No. 1 382 014 (Kendall Co.) and U.S. Patent No. 4 023 607 (Jeusen) it is difficult to seal the inlet and/or the outlet tube to the superposed bag walls.

One suggestion that has been made is to weld or otherwise attach a connection having a flat flange to the face of a bag wall (see U.S. Patents Nos. 3 650 272 Ericson and 3 568 965 Clark), rather than attempting to fix a tube between the superposed marginal edge regions of the two bag walls. In the '272 Ericson U.S. Patent a face-to-face connection is envisaged between flat surfaces of spouts 33, 34 and one wall 11 of the bag. In the present Applicant's U.K. Application No. (GB-A) 2 080 247 a handle unit and an outlet unit are attached in face-to-face manner to a wall of a bag, for example by welding.

An aim of the present invention is to simplify and improve the manufacture of drainage bags so as to reduce the number of manufacturing steps needed and to reduce the number of bags produced which have to be rejected upon test because of leakage problems. It is also an aim of the invention to produce a bag which is versatile and to which inlet and outlet tubes can readily be attached after rather than during the welding steps of the manufacture.

According to the present invention, there is provided a method of manufacturing a urine drainage bag which includes the steps of:

(a) placing a handle and inlet spout unit and an outlet unit in a welding machine so that respective flat surfaces thereof are uppermost;

(b) placing first and second layers of plastics material over the handle and inlet spout unit, the first layer being intended to form one wall of a completed bag and the second layer being intended to form part of an internal flap valve;

(c) welding in one step the first and second layers to the flat surface of the handle and inlet spout unit and the first layer only to the flat surface of the outlet unit;

(d) placing two more layers upon the assembly so produced, the third layer being intended to form part of the flap valve and the fourth layer being intended to form the other wall of the bag;

(e) making as a second welding step in the same welding machine, a complete peripheral weld to join together the periphery of the first and fourth layers; and

(f) either prior to or simultaneously with the step (c), providing a hole for exit of urine in the first layer and a hole for ingress of urine through the first and second layers.

According to a preferred method in accordance with the invention, the spout of the combined handle and spout unit is attached in a separate step after the welding operations are completed to a length of inlet tubing.

A further advantageous but optional feature of the invention is that the outlet unit is of the kind wherein a tube is bent up and plugged onto a fixed plug in order to close the outlet. Such valves are known *per se* (see British Patent 1 024 661 and Published U.K. specification 2 058 011) but its use in this context is particularly effective in that a flat flange surface can readily be welded to one bag wall in the manner aforesaid.

An important advantage of the design particularly disclosed and illustrated herein is that it can be employed equally well with urine bags made of PVC based plastics materials and polyolefine based plastics material. For the former, a radio frequency welding procedure would preferably be employed, whereas for the latter, a heat sealing procedure is preferred. Another important advantage is that only one welding step is utilized to connect the outlet unit and the combined handle and inlet spout to one of the bag walls, and that this weld is made entirely independently of the weld which closes the outer periphery of the bag. In consequence, it is less difficult to ensure that both of these welds are made in a manner which is liquid tight and, as a consequence, the incidence of unsatisfactory bags which are rejected upon test due to leakage is reduced.

The inlet spout forming part of the combined handle and inlet unit may readily be connected as a separate operation to an inlet tube, which means that the material of the tube does not have to be compatible from the point of view of plastics welding with the material of the inlet spout. Also, fabricating the handle and the inlet spout unit as a single integral piece of plastics material is highly advantageous in that it can be cheaply and easily produced and can be formed of a shape such that it can be hung up in a number of different ways and includes a clip for guiding and holding an inlet tube. A preferred material for the combined handle and inlet spout unit is high density polythene. Alternatively, a rigid PVC material may be used.

Because the inlet tube is not made integral with the bag, in contrast to current commercial designs on the market, the machine manufacturing the bags can be designed to run very fast. This is because the free length of tube (usually around 1 meter or 3 or 4 feet long) when it is integral with the bag, represents a serious handling problem during high speed manufacture. The design according to the present disclosure is well able to tolerate a manufacturing rate of over 1500 and up to 2000 or more bags an hour. In addition, it is advantageous to attach the inlet tube separately later, either by hand or by machine, because there is a saving of tube material in the event that a bag is found to be faulty and is scrapped. Under the conventional method with an integral inlet tube, the tube material had to be reclaimed in this event.

The invention will be better understood from the following non-limiting description of an example thereof given with reference to the accompanying drawings in which:

Fig. 1 is a front view of one example of urine drainage bag according to the invention, and

Fig. 2 is a vertical central cross section taken on the line A—A of Fig. 1.

The illustrated urine drainage bag 10 includes first and second walls 12, 14 between which are disposed layers 16 and 18 of plastics material which together form an internal flap valve of conventional kind. The layers 12 and 14 are welded together around the periphery of a weld 20 to define the bag 10.

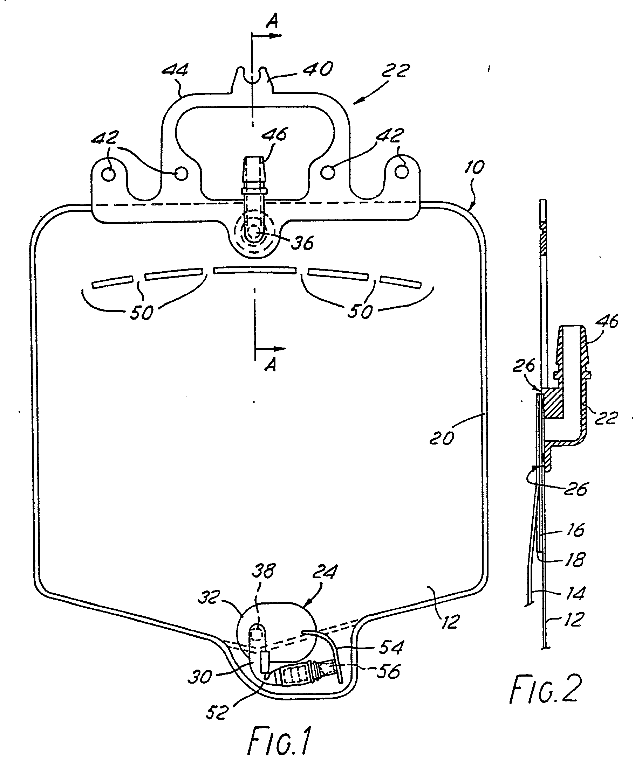

The bag 10 also includes an integral handle and spout unit 22 and an outlet unit 24. The unit 22 has a flat surface 26 to which, during manufacture, the bag wall 12 and the layer 16 are welded in a single welding operation. For this purpose, the combined handle and spout unit is placed within a plastics welding machine with the surface 26 upwardly, und the layers 12 and 16 are laid thereover in the relative positioning illustrated in Figs. 1 and 2.

In manufacture, in the same welding step, the outlet unit 24 which has a spout 30 and a flange 32, is laid within the welding machine with the flat flange surface facing upwardly. It will be seen that as the layer 16 does not extend down to the bottom of the bag, in the same welding operation just described above the flange 32 of the outlet unit is welded to the bag wall 12. Prior to or simultaneous with this welding operation, a hole 38 is punched or otherwise made in the bag wall 12 and a hole 36 is made through the bag wall 12 and the layer 16. These holes are to allow ingress of urine to the interior of the bag, in the case of holes 36 and exit of urine in the case of hole 38.

The combined handle and spout unit 22 has a tube clip portion 40 and holes 42, as well as a handle 44. The holes 42 are to permit the bag to be hung up from suitably spaced hooks. The clip or tab 40 is useful to support and guide an inlet tube (not shown) connected in use to the spout 46.

According to an advantageous but not essential feature of the invention, a series of bar welds 48 may be located across the bag in the region of the internal flap valve, these welds welding all 4 layers. The purpose of this is to provide an obstruction which guides urine entering the bag to be distributed across its width before it falls to the lower part of the bag. It will be realized that the urine in passing to the lower region of the bag passes through the spaces 50 between the welds.

The illustrated outlet tap is one which has a flexible tube which can be bent as shown at 52, there being a flexible strip 54 integral with the flange 32, the strip 54 carrying at its end a plug 56 which can be inserted in the open end of the tube 30 to close it off. Other designs of tap, for example the one disclosed and claimed in British Patent Application No. 2 101 .274.

The method of making a urine bag as illustrated in Figs. 1 and 2 includes the steps of:

(a) placing the handle and inlet spout unit 22 and the outlet unit 24 in a welding machine so that the respective flat surfaces 26 and the rear surface of flange 32 (as illustrated) are uppermost,

(b) placing first and second layers 12 and 16 of plastics material over the handle and inlet spout unit 22,

(c) welding in one welding operation the layers 12 and 16 to the flat surface 26 and the first layer 12 only to the rear surface of the flange 32,

(d) placing a plastics layer 18 and the bag wall 14 upon the assembly so produced,

(e) making, as a second welding step in the same welding machine, a complete peripheral weld 20 to join together the periphery of the bag walls 12 and 14, that is to say the periphery of the first and fourth layers. In this operation the internal flap valve layers 16 and 18 are joined together around their top and side margins, and

(f) either prior to or simultaneously with the step (c), providing a hole 38 for exit of urine in the first layer 12 and a hole 36 for ingress of urine through the first and second layers 12 and 16.

**Claims**

1. A method of manufacturing a urine drainage bag (10) which includes the steps of:

(a) placing a handle and inlet spout unit (22) and an outlet unit (24) in a welding machine so that respective flat surfaces thereof are uppermost;

(b) placing first and second layers (12, 16) of plastics material over the handle and inlet spout unit (22), the first layer (12) being intended to form one wall of a completed bag and the second layer (16) being intended to form part of an internal flap valve;

(c) welding in one step the first and second layers (12, 16) to the flat surface of the handle and inlet spout unit (22) and the first layer (12) only to the flat surface of the outlet unit (24);

(d) placing two more layers (18, 14) upon the assembly so produced, the third layer (18) being intended to form part of the flap valve and the fourth layer (14) being intended to form the other wall of the bag;

(e) making as a second welding step in the same welding machine, a complete peripheral weld (20) to join together the periphery of the first and fourth layers (12, 14); and

(f) either prior to or simultaneously with the step (c), providing a hole (38) for exit of urine in the first layer (12) and a hole (36) for ingress of urine through the first and second layers (12 and 16).

2. A method according to Claim 1, in which the spout (46) of the combined handle and spout unit (22) is attached in a separate step after the welding operations are completed to a length of inlet tubing.

3. A method according to Claim 1 or 2 in which the outlet unit is of the kind wherein a tube is bent up and plugged onto a fixed plug (56) in order to close the outlet.

## Patentansprüche

1. Verfahren zum Herstellen eines Drainagebeutels (10) für Urin, das die Stufen aufweist:

(a) Anordnen eines Handgriffs und einer Einlaßrohreinheit (22) und einer Auslaßeinheit (24) in einer Schweißmaschine, so daß deren jeweilige flache Flächen zuoberst sind,

(b) Anordnen von ersten und zweiten Lagen (12, 16) aus Kunststoffmaterial über den Handgriff und die Einlaßtrichtereinheit (22), wobei die erste Lage (12) eine Wand eines vollständigen Beutels bilden soll und die zweite Lage (16) ein Teil eines inneren Klappenventils bilden soll,

(c) Schweißen in einer Stufe der ersten und zweiten Lage (12, 16) an der ebenen Fläche des Handgriffs und der Einlaßtrichtereinheit (22) und der ersten Lage (12) nur an der ebenen Fläche der Auslaßeinheit (24),

(d) Anordnen von zwei weiteren Lagen (18, 14) auf der so hergestellten Anordnung, wobei die dritte Lage (18) einen Teil des Klappenventils bilden soll und die vierte Lage (14) die andere Wand des Beutels bilden soll.

(e) Herstellen als einen zweiten Schweißschritt in der gleichen Schweißmaschine einer vollständigen Randschweißung (20), so daß der Rand der ersten und vierten Lage (12, 14) miteinander verbunden werden, und

(f) entweder vor oder gleichzeitig mit der Stufe (c) Bereitstellen eines Lochs (38) für den Austritt von Urin in der ersten Lage (12) und eines Lochs (36) für den Eintritt von Urin durch die erste und zweite Lage (12 und 16).

2. Verfahren nach Anspruch 1, bei dem der Trichter (46) der Kombination aus Handgriff und Trichtereinheit (22) in einer getrennten Stufe angebracht wird, nachdem die Schweißvorgänge bis zu einer Länge des Einlaßrohres abgeschlossen sind.

3. Verfahren nach Anspruch 1 oder 2, bei dem die Auslaßeinheit eine solche ist, bei ein Rohr abgeknickt und an einem festen Stopfen (56) zum Verschließen des Auslasses aufgesteckt wird.

## Revendications

1. Procédé de fabrication d'une poche de drainage d'urine (10) comportant les étapes suivantes:

(a) mise en place d'un ensemble de poignée et de bec d'arrivée (22) et d'un ensemble de sortie (24) dans une machine de soudage, de sorte que leurs surfaces plates respectives se trouvent sur le dessus;

(b) mise en place d'une première et d'une seconde couche (12, 16) de matière plastique pardessus l'ensemble poignée et bec d'arrivée (22), la première couche (12) étant destinée à former l'une des parois d'une poche complète et la seconde couche (16) étant destinée à faire partie d'une soupape à clapet interne;

(c) soudage, en une étape, de la première et de la seconde couche (12, 16) à la surface plate de l'ensemble poignée et bec d'arrivée (22), et de la première couche (12) seulement à la surface plate de l'ensemble de sortie (24);

(d) mise en place de deux couches supplémentaires (18, 14) sur l'ensemble ainsi produit, la troisième couche (18) étant destinée à faire partie de la soupape à clapet et la quatrième couche (14) étant destinée à former l'autre paroi de la poche;

(e) réalisation, dans une seconde étape de soudage, dans la même machine de soudage, d'une soudure périphérique complète (20) pour réunir l'une à l'autre la première et la quatrième couche (12, 14) par leur pourtour; et

(f) soit avant soit en même temps que l'étape (c), perçage d'un trou (38) permettant la sortie de l'urine dans la première couche (12), et d'un trou (36) permettant l'arrivée de l'urine à travers la première et la seconde couche (12 et 16).

2. Procédé selon la revendication 1, dans lequel le bec (46) de l'ensemble (22) formé par la poignée et le bec d'arrivée est fixé, dans une étape distincte, après la fin des opérations de soudage, à une longueur de tube d'arrivée.

3. Procédé selon la revendication 1 ou 2, dans lequel l'ensemble de sortie est du type dans lequel un tube est coudé et enfoncé dans un bouchon fixe (56) afin de fermer la sortie.

EP 0 147 048 B1

FIG.1

FIG.2